# EUROPÄISCHE PATENTSCHRIFT

(11) **EP 0 590 382 B1**
(45) Veröffentlichungstag und Bekanntmachung des Hinweises auf die Patenterteilung: **24.07.1996**
(21) Anmeldenummer: 93114539.5
(22) Anmeldetag: 10.09.1993
(51) Int. Cl.: A61K 9/16, A61K 31/135

(54) **Verfahren zur Herstellung von Pellets aus einem Ephedrinderivat**
Method to produce pellets containing an ephedrine derivative
Procédé pour la production de granules contenant un dérivé de l'éphédrine

(30) Priorität: 21.09.1992 DE 4231493
(43) Veröffentlichungstag der Anmeldung: 06.04.1994
(73) Patentinhaber: NORDMARK ARZNEIMITTEL GmbH., D-25436 Uetersen (DE)
(72) Erfinder: Moest, Thomas, Dr., D-2082 Moorrege (DE); Loeffler, Uwe, Dr., D-2082 Uetersen (DE); Waiblinger, Hans, Dr., D-4970 Bad Oeynhausen (DE)
(74) Vertreter: Karau, Wolfgang, Dr.

(56) Entgegenhaltungen:
- EP-A- 0 418 600
- CHEMICAL ABSTRACTS, no. 2, 25. Januar 1960, Columbus, Ohio, US; abstract no. 96979f, SHIBA, MOTOHARU, ET AL. 'enteric coating of drug with hydroxypropylmethylcellulose phtalate'

## Beschreibung

Die Erfindung betrifft ein einfaches Verfahren zur Herstellung von Pellets mit ausgeprägt kugelförmiger Struktur, die vorzugsweise zu 100 % aus Ephedrin-Derivaten bestehen, deren Retardierung und daraus hergestellte Arzneimittel.

Zum Aufbau von Pellets aus Ephedrin-Derivaten nach herkömmlichen Technologien werden Nonpareilles als Starterkerne zum Aufdragieren oder Klebemittel und/oder andere Hilfsstoffe zur Herstellung einer plastisch verformbaren Masse benötigt.

Diese Herstellungverfahren sind aufwendig, und sie erlauben nicht die Herstellung von Pellets aus Ephedrin-Derivaten mit maximaler, d.h. 100 %, Wirkstoffkonzentration; die Wirkstoffdichte ist also begrenzt. Eine hohe Wirkstoffdichte ist jedoch erforderlich, um höhere Mengen an Ephedrin-Derivaten z.B. in Hartgelatine-Kapseln kleiner Größe abfüllen zu können, die angenehm zu schlucken sind.

Ephedrin-Derivate in kommerziell erhältlicher Qualität und Feinheit neigen ferner während der Lagerung zur Aggregatbildung sowie, im Falle mikronisierter Ware, zur Staubentwicklung und sind demzufolge schwierig zu handhaben.

Aus US 5 160 469 ist bekannt, daß das nahezu wasserunlösliche Xanthin-Derivat Theophyllin zu weitgehend kugelförmigen Pellets agglomeriert werden kann. Wendet man das in diesem Patent beschriebene Verfahren auf Ephedrin-Derivate an, so werden aufgrund der hohen Wasserlöslichkeit der Ephedrin-Derivate keine Pellets, sondern vielmehr ein Zweiphasengemisch erhalten.

Der Erfindung lag die Aufgabe zugrunde, auf einfache Weise kugelförmige Pellets aus Ephedrin-Derivaten mit hoher Wirkstoffkonzentration herzustellen, die zugleich eine hohe Schüttdichte von mehr als 0,5 g/cm³ und hohe Abriebfestigkeit aufweisen, und somit einerseits eine möglichst hohe Wirkstoffdichte, z.B. in Kapseln, ermöglichen und andererseits optimal handhabbar sind.

Die Lösung dieser Aufgabe besteht in einem verfahren zur Herstellung von Pellets mit ausgeprägt kugeliger Struktur, einer Korngröße im Bereich von 0,1 bis 4 mm und einer Schüttdichte über 0,5 g/cm³, die zu 90 bis 100 Gew.-% aus einem Ephedrin-Derivat und zu 0 bis 10 Gew.-% aus einem galenischen Hilfsmittel bestehen, das dadurch gekennzeichnet ist, daß man Ephedrin-Derivat-Pulver mit einer mittleren Korngröße von 0,5 bis 50 µm bei 0 bis 90°C unter Rühren in einer nichtlösenden, mit Wasser nicht mischbaren Flüssigkeit mit einem Siedepunkt im Bereich von 60 bis 160°C suspendiert, 5 bis 60 Gew.-%, bezogen auf das Ephedrin-Derivat, einer konzentrierte wäßrige Lösung des jeweiligen Ephedrin-Derivates als Agglomerationsflüssigkeit unter weiterem Rühren zusetzt, um 5 bis 40 K pro Stunde auf -5 bis 25°C abkühlen läßt, falls vorher erwärmt worden war, wobei man die Rührgeschwindigkeit nach der Agglomeration der Pulverteilchen auf einen für die gewünschte mittlere Pelletgröße erforderlichen Wert einstellt, die entstandenen Pellets trennt und trocknet.

Als Ephedrin-Derivate im Sinn der Erfindung gelten z.B. DL-Methylephedrin-Hydrochlorid, Pseudoephedrin-Hydrochlorid sowie Pseudoephedrin-Sulfat.

Man geht aus von einem möglichst feinkörnigen, "mikronisierten" Ephedrin-Derivat-Pulver. Die mittlere Korngröße sollte im Bereich von 0,5 bis 50, vorzugsweise 1 bis 20 µm liegen.

Im einzelnen wird folgendermaßen verfahren:
Man dispergiert das Ephedrin-Derivat-Pulver in der Flüssigkeit unter stetigem Rühren im beginnenden turbulenten Bereich, wobei es keinen Einfluß auf das Ergebnis hat, ob man das Pulver vorlegt und die Flüssigkeit dazugießt oder umgekehrt. Grenzflächenspannungsverändernde Stoffe sind nicht erforderlich. Die Temperatur der Flüssigkeit liegt dabei im Bereich von 0 bis 90, vorzugsweise 50 bis 65°C. Nach Zusatz, gleichgültig ob langsam oder rasch, tropfenweise oder in einem Guß, von 5 bis 60, vorzugsweise 25 bis 45 Gew.-%, bezogen auf das Ephedrin-Derivat, der Agglomerationsflüssigkeit, die in der bevorzugten Ausführungsform aus einer konzentrierten, insbesondere (nahezu) gesättigten wäßrigen Lösung des Ephedrin-Derivates besteht, läßt man die Temperatur pro Stunde um 5 bis 40 K absinken. Nachdem die zunächst trübe Dispersion des Pulvers sich durch Agglomeration geklärt hat, stellt man die (in Vorversuchen ermittelte) zur Erzielung der gewünschten Korngröße (mittlere Pelletgröße) erforderliche Rührgeschwindigkeit ein. Je nach der Menge der zugegebenen Agglomerationsflüssigkeit und der Rührerdrehzahl kann die mittlere Korngröße der Pellets im Bereich von 0,1 bis 4 mm, vorzugsweise 0,2 bis 3 mm eingestellt werden. Die Korngrößenverteilung ist innerhalb dieses Bereiches in der Regel eng, die Teilchen sind gleichmäßig kugelförmig. Sie werden nach dem Abkühlen auf -5 bis +25°C, zweckmäßig auf Raumtemperatur, von der Flüssigkeit getrennt, ggf. mit einem leicht siedenden, nicht lösenden Lösungsmittel gewaschen und in üblicher Weise (vgl. Lehrbücher der Pharmazeutischen Technologie) bei 40 bis 80°C getrocknet. Ihre Schüttdichte beträgt mehr als 0,5 g/cm³. Sie sind in hohem Maße abriebfest. In einem Gerät zur Bestimmung der mechanischen Abriebfestigkeit (Born Friabimat SA 400, Fa. Born Gerätebau, D-3554 Gladenbach) wurde nach der sehr hohen Schüttelbelastung von 990 min⁻¹ und 180 sec Dauer nur ein Abrieb von maximal 5 % ermittelt.

Die Mengenverhältnisse Ephedrin-Derivat : nicht lösender, mit Wasser nicht mischbarer Flüssigkeit : wäßriger Lösung liegen im Bereich von 1 kg : 4 bis 25 l : 0,05 bis 0,6 l.

Das Verfahren kann auch kontinuierlich durchgeführt werden.

Bei einer (weniger bevorzugten) Variante des Verfahrens wird nicht erwärmt, sondern von vornherein bei 0 bis 40°C gearbeitet, und als Agglomerationsflüssigkeit werden 5 bis 20 Gew.-%, bezogen auf das Ephedrin-Derivat, Wasser eingesetzt. Im übrigen verfährt man genauso wie oben beschrieben. Bei einer weiteren, noch weniger bevorzugten Verfahrensvariante wird als Agglomerationsflüssigkeit ein polares organisches Lösungsmittel eingesetzt, das beim Vermischen mit der Suspensionsflüssigkeit eine Emulsion bildet, die mindestens so lange stabil ist, bis die Agglomeration der Pulverteilchen (in der Regel innerhalb weniger Sekunden) erfolgt ist. Beispiele dafür sind Methanol, Ethanol, Propanol, Essigsäureester, Aceton, halogenierte Kohlenwasserstoffe und aromatische Kohlenwasserstoffe.

Die Pellets bestehen vorzugsweise zu 100 % aus dem Wirkstoff. Sie können im Prinzip aber auch bis zu 2 oder 5 oder gar 10 Gew.-% üblicher galenischer Hilfsmittel enthalten. Diese können im Gemisch mit dem Ephedrin-Derivat-Pulver eingesetzt und zusammen mit diesem agglomeriert werden, oder sie werden in der Suspensionsflüssigkeit oder der Agglomerationsflüssigkeit gelöst und daraus an die Pulverteilchen adsorbiert und somit in die Agglomerate integriert.

Die Pellets können direkt oder nach dem Überziehen mit einem retardierenden (und/oder magensaftresistenten) Lack in Steckkapseln oder in Dosierbeutel abgefüllt werden. Retardierende Lacke enthalten die für diesen Zweck üblichen, in wäßrigem Medium unlöslichen, diffusionskontrollierenden Polymeren, wie sie in den galenischen Lehr- und Handbüchern beschrieben sind, z.B. Polymethacrylate, Zellulosederivate, Vinylpolymere. Bedingt durch die hohe mechanische Festigkeit, die ausgeprägt kugelige Gestalt und die gleichmäßige Oberfläche der Pellets ist es möglich, eine kontrollierte Wirkstofffreisetzung mit sehr geringen Lackmengen in beliebigen Coatinggeräten auf einfache Weise zu erhalten. Die gleichmäßig kugelförmige Struktur der Pellets ermöglicht ein präzises Füllen von Kapseln in dichtester Kugelpackung und daher Kapseln mit hohem Wirkstoffgehalt.

Feste Arzneiformen im Sinne der Erfindung, die die erfindungsgemäß hergestellten Pellets enthalten, sind Steckkapseln, Dosierbeutel sowie Tabletten, die aus den Pellets unter Verwendung von bis zu 50 vorzugsweise bis zu 10 Gew.-%, bezogen auf die fertige Tablette, Bindemitteln und/oder anderen üblichen galenischen Hilfsstoffen wie Füllstoffen, Tablettensprengmitteln, Retardierungsmitteln, Gleitmitteln, Farbstoffen gepreßt werden.

### Beispiel 1

In einem 1 l-Doppelmantelgefäß mit Schrägblattrührer und Stromstörern wurden 500 ml n-Hexan auf 2°C unter Rühren abgekühlt. Nach Zugabe von 25 g mikronisiertem Pseudoephedrin-Hydrochlorid wurde mit einer Umfangsgeschwindigkeit von 3,2 m/s suspendiert. 7 ml einer bei 2°C gesättigten wäßrigen Pseudoephedrin-Hydrochlorid-Lösung wurden in die Suspension eingerührt. Nach Reduzieren der Umfangsgeschwindigkeit auf 1,3 m/s nach der Agglomeration der suspendierten Teilchen und langsamem Abkühlen mit ca. 10 K/h auf -3°C wurden die entstandenen Pellets mit einer Nutsche separiert. Getrocknet wurde in einem geeigneten Trockenschrank bei ca. 50°C.

Die Pellets wiesen ein Kornband von 200 µm bis 1600 µm auf, ein ausgeprägtes Maximum lag bei 720 µm.

Die Schüttdichte nach DIN 53 468 betrug 0,51 g/cm³.

Der Abrieb nach 180 sec im Friabimat SA 400 bei 990 min⁻¹ betrug weniger als 3,9 %.

### Beispiel 2

In einem 3 l-Doppelmantelgefäß mit Schrägblattrührer und Stromstörern wurden 2,5 l n-Hexan auf 45°C unter Rühren erwärmt. Nach Zugabe von 0,25 kg mikronisiertem Pseudoephedrin-Hydrochlorid wurde mit einer Umfangsgeschwindigkeit von 4,4 m/s suspendiert. 0,1 l einer bei 45°C gesättigten wäßrigen Pseudoephedrin-Hydrochlorid-Lösung wurden auf 65°C erwärmt und in die Suspension eingerührt. Nach Reduzieren der Umfangsgeschwindigkeit auf 1,7 m/s nach der Agglomeration der suspendierten Teilchen und langsamem Abkühlen mit ca. 10 K/h auf 20°C wurden die entstandenen Pellets mit einer Nutsche separiert. Getrocknet wurde in einem geeigneten (explosionsgeschützten) Trockenschrank bei ca. 50°C.

Die Pellets wiesen ein Kornband von 300 bis 1600 µm auf, ein ausgeprägtes Maximum lag bei 800 µm.

Die Schüttdichte nach DIN 53 468 betrug 0,52 g/cm³.

Der Abrieb nach 180 sec im Friabimat SA 400 bei 990 min⁻¹ betrug weniger als 1,5 %.

### Beispiel 3

In einem 3 l-Doppelmantelgefäß mit Schrägblattrührer und Stromstörern wurden 2500 ml n-Hexan auf 50°C unter Rühren erwärmt. Nach Zugabe von 0,125 kg mikronisiertem Pseudoephedrin-Sulfat wurde mit einer Umfangsgeschwindigkeit von 3,2 m/s suspendiert. 55 ml einer bei 50°C halbgesättigten, wäßrigen Pseudoephedrin-Sulfat-Lösung wurden in die Suspension eingerührt. Nach Reduzieren der Umfangsgeschwindigkeit auf 1,7 m/s nach der Agglomeration der suspendierten Teilchen und Abkühlen mit ca. 25 K/h auf 20°C wurden die entstandenen Pellets mit einer Nutsche separiert. Getrocknet wurde in einem geeigneten explosionsgeschützten Trockenschrank bei ca. 50°C.

Die Pellets wiesen ein Kornband von 600 bis 2000 µm auf, ein ausgeprägtes Maximum lag bei 1000 µm.

Die Schüttdichte nach DIN 53 468 betrug 0,54 g/cm³.

Der Abrieb nach 180 sec im Friabimat SA 400 bei 990 min⁻¹ betrug weniger als 2,5 %.

### Beispiel 4

In einem 1 l-Gefäß mit Schrägblattrührer wurden 500 ml n-Hexan bei Raumtemperatur gerührt. Nach Zugabe von 0,1 kg mikronisiertem DL-Methylephedrin·HCl wurde mit einer Umfangsgeschwindigkeit von 2,6 m/s suspendiert. 45 ml einer bei Raumtemperatur gesättigten, wäßrigen DL-Methylephedrin·HCl-Lösung wurden in die Suspension eingerührt. Nach Erhöhen der Umfangsgeschwindigkeit auf 3,4 m/s nach der Agglomeration der suspendierten Teilchen und 10-minütigem Rühren wurden die entstandenen Pellets mit einer Nutsche separiert. Getrocknet wurde in einem geeigneten Trockenschrank bei ca. 50°C.

Die Pellets wiesen ein Kornband von 100 µm bis 4000 µm auf, ein ausgeprägtes Maximum lag bei 3000 µm.

Die Schüttdichte nach DIN 53 468 betrug 0,56 g/cm³.

Der Abrieb nach 180 sec im Friabimat SA 400 bei 990 min⁻¹ betrug weniger als 4,4 %.

## Patentansprüche

1. Verfahren zur Herstellung von Pellets mit ausgeprägt kugeliger Struktur, einer Korngröße im Bereich von 0,1 bis 4 mm und einer Schüttdichte über 0,5 g/cm³, die zu 90 bis 100 Gew.-% aus einem Ephedrin-Derivat und zu 0 bis 10 Gew.-% aus einem galenischen Hilfsmittel bestehen, dadurch gekennzeichnet, daß man Ephedrin-Derivat-Pulver mit einer mittleren Korngröße von 0,5 bis 50 µm bei 0 bis 90°C unter Rühren in einer nichtlösenden, mit Wasser nicht mischbaren Flüssigkeit mit einem Siedepunkt im Bereich von 60 bis 160°C suspendiert, 5 bis 60 Gew.-%, bezogen auf das Ephedrin-Derivat, einer konzentrierten wäßrigen Lösung des jeweiligen Ephedrin-Derivates als Agglomerationsflüssigkeit unter weiterem Rühren zusetzt, um 5 bis 40 K pro Stunde auf -5 bis 25°C abkühlen läßt, falls vorher erwärmt worden war, wobei man die Rührgeschwindigkeit nach der Agglomeration der Pulverteilchen auf einen für die gewünschte mittlere Pelletgröße erforderlichen Wert einstellt, die entstandenen Pellets trennt und trocknet.

2. Verfahren nach Anspruch 1, dadurch gekennzeichnet, daß man die Mengenverhältnisse Ephedrin-Derivat zu nichtlösender, mit Wasser nicht mischbarer Suspensionsflüssigkeit zu Agglomerationsflüssigkeit im Bereich von 1 kg : 4 bis 25 l : 0,05 bis 0,6 l wählt.

3. Verfahren zur Herstellung von Pellets nach einem der Ansprüche 1 bis 2, dadurch gekennzeichnet, daß als Ephedrin-Derivat DL-Methylephedrin-Hydrochlorid, Pseudoephedrin-Hydrochlorid oder Pseudoephedrin-Sulfat eingesetzt wird.

4. Verfahren zur Herstellung von Retardpellets durch Überziehen von Ephedrin-Derivat-Pellets, die nach einem der Ansprüche 1 bis 3 hergestellt wurden, mit mindestens einem diffusionskontrollierenden, in wäßrigem Medium nicht-löslichen Polymeren.

5. Feste Arzneiform zur peroralen Anwendung, die, ggf. neben üblichen galenischen Hilfsmitteln, 50 bis 100 Gew.-% der nach einem der Ansprüche 1 bis 3 hergestellten Ephedrin-Derivat-Pellets oder nach Anspruch 4 erhaltene Retardpellets oder Mischungen von beiden als Wirkstoff enthält.

## Claims

1. A process for the production of pellets with a markedly spherical structure, a particle size in the range from 0.1 to 4 mm and an apparent density above 0.5 g/cm³, which comprise 90 - 100% by weight of an ephedrine derivative and 0 - 10% by weight of a pharmaceutical auxiliary, which comprises suspending ephedrine derivative powder with an average particle size of from 0.5 to 50 µm at 0 to 90°C with stirring in a liquid nonsolvent which is immiscible with water and has a boiling point in the range from 60 to 160°C, adding from 5 to 60% by weight, based on the ephedrine derivative, of a concentrated aqueous solution of the particular ephedrine derivative as agglomeration liquid with further stirring, allowing to cool at 5 to 40 K per hour to -5 to 25°C if previous heating has been carried out, with the stirring rate being adjusted after the agglomeration of the powder particles to a value which is necessary for the required average pellet size, and separating and drying the resulting pellets.

2. A process as claimed in claim 1, wherein the ratios of the amounts of ephedrine derivative to suspending liquid nonsolvent which is immiscible with water to agglomeration liquid is chosen in the range of 1 kg : 4 to 25 l : 0.05 to 0.6 l.

3. A process for the production of pellets as claimed in either of claims 1 and 2, wherein DL-methylephedrine hydrochloride, pseudoephedrine hydrochloride or pseudoephedrine sulfate is used as ephedrine derivative.

4. A process for the production of slow-release pellets by coating ephedrine derivative pellets, which have been produced as claimed in any of claims 1 to 3, with at least one diffusion-controlling polymer which is insoluble in aqueous medium.

5. A solid drug form for oral use which, besides conventional pharmaceutical auxiliaries where appropriate, contains from 50 to 100% by weight of the ephedrine derivative pellets produced as claimed in any of claims 1 to 3, or slow-release pellets obtained as claimed in claim 4, or mixtures of the two, as active ingredient.

## Revendications

1. Procédé pour la préparation de pellets ayant une structure parfaitement sphérique, une taille de particules dans l'intervalle de 0,1 à 4 mm et une masse volumique apparente de 0,5 g/cm³, qui consistent en 90 à 100 % en poids d'un dérivé d'éphédrine et en 0 à 10 % en poids d'un adjuvant galénique, caractérisé par le fait qu'on met en suspension de la poudre de dérivé d'éphédrine ayant une taille moyenne de particules de 0,5 à 50 µm entre 0 et 90°C sous agitation dans un liquide non dissolvant, non miscible avec l'eau, bouillant dans l'intervalle de 60 à 160°C, on ajoute 5 à 60 % en poids, par rapport au dérivé d'éphédrine, d'une solution aqueuse concentrée du dérivé d'éphédrine concerné comme liquide d'agglomération, en poursuivant l'agitation, on laisse refroidir de 5 à 40 K par heure jusqu'à -5 à 25°C, dans le cas où il y a eu chauffage au préalable, tandis que l'on ajuste la vitesse d'agitation après l'agglomération des particules de poudre à une valeur nécessaire pour la taille moyenne souhaitée des pellets, on sépare les pellets formés et on sèche.

2. Procédé selon la revendication 1, caractérisé par le fait qu'on choisit le rapport pondéral du dérivé d'éphédrine au liquide de suspension non dissolvant, non miscible avec l'eau, et au liquide d'agglomération dans l'intervalle de 1 kg : 4 à 25 l : 0,05 à 0,6 l.

3. Procédé pour la préparation de pellets selon l'une des revendications 1 et 2, caractérisé par le fait qu'on emploie, comme dérivé d'éphédrine, du chlorhydrate de DL-méthyléphédrine, du chlorhydrate de pseudoéphédrine ou du sulfate de pseudoéphédrine.

4. Procédé pour la préparation de pellets à effet retard par revêtement de pellets de dérivé d'éphédrine qui ont été préparés selon l'une quelconque des revendications 1 à 3, avec au moins un polymère réglant la diffusion, non soluble en milieu aqueux.

5. Forme médicamenteuse solide pour administration perorale contenant, outre des adjuvants galéniques classiques, 50 à 100 % en poids des pellets de dérivé d'éphédrine préparés selon l'une quelconque des revendications 1 à 3 ou de pellets à effet retard préparés selon la revendication 4 ou de mélanges des deux comme principe actif.
